# EUROPEAN PATENT SPECIFICATION

(11) **EP 1 889 591 B1**
(45) Date of publication and mention of the grant of the patent: **01.03.2017**
(21) Application number: 06732261.0
(22) Date of filing: 24.04.2006
(51) Int. Cl.: A61H 3/00

(54) **THIGH PART MOUNTING DEVICE FOR WALKING ASSISTING DEVICE**
VORRICHTUNG ZUR BEFESTIGUNG EINES OBERSCHENKELTEILS FÜR EINE GEHHILFEVORRICHTUNG
DISPOSITIF DE MONTAGE DE PARTIE CUISSE POUR DISPOSITIF D'AIDE A LA MARCHE

(30) Priority: 17.05.2005 JP 2005143422
(43) Date of publication of application: 20.02.2008
(73) Proprietor: HONDA MOTOR CO., LTD., Tokyo 107-8556 (JP)
(72) Inventor: HIRATA, Takashi, Wako-shi, Saitama 351-0193 (JP); SHIMADA, Kei, Wako-shi, Saitama 351-0193 (JP); FUJII, Takako, Kyoto-shi, Kyoto 601-8530 (JP)
(74) Representative: Hague, Alison Jane
(86) International application number: PCT/JP2006/308550
(87) International publication number: WO 2006/123514

(56) References cited:
- WO-A1-03/091014
- JP-A- 2002 301 124
- JP-A- 2003 220 102
- JP-A- 2003 220 102
- JP-C1- 6 380
- US-A- 3 552 044
- US-B1- 6 197 099

## Description

### TECHNICAL FIELD

The present application claims priority from Japanese application No. 2005-143422 filed May 17, 2005.

The present invention relates to a walking assistance device provided with a power actuator for providing an assist force for assisting the muscular force of a person having a deficiency in the functions of his lower limbs, and in particular to a walking assistance device equipped with a femoral support member that can secure a free end of an assist force transmitting arm for transmitting an assist force of the power actuator to a femoral region of a wearer without causing any discomfort.

### BACKGROUND OF THE INVENTION

Various walking assistance devices have been proposed (see Japanese patent laid open publication No. 07-163607, for instance) for the purpose of providing an assistance to a person who has a difficulty in walking by himself owing to the lack of muscular force, and such a device typically includes a power actuator such as an electric motor fitted on the wearer near his hip joint or knee joint to assist the movement of the lower limbs of the wearer.

In the walking assistance device disclosed in Japanese patent laid open publication No. 07-163607, a femoral support member is worn around a femoral region of the wearer, and an actuating force of the power actuator is transmitted to the femoral region of the wearer via the femoral support member. The femoral support member is provided with a cylindrical shape so as to surround the femoral region and be firmly secured thereto, and is made of soft and flexible material such as elastic fabric, leather and plastic sheet. The femoral support member is configured to be opened at a part thereof so as to be detachably fitted around the femoral region.

### BRIEF SUMMARY OF THE INVENTION

### TASKS TO BE ACCOMPLISHED BY THE INVENTION

However, according to the structure disclosed in Japanese patent laid open publication No. 07-163607, because the femoral support member is made of soft material and has a low rigidity, the actuating force of the power actuator tends to concentrate in the joint between the power transmitting arm and femoral support member, and this prevents the actuating force from being transmitted evenly to the entire femoral region. For this reason, some discomfort was inevitable for the wearer because of a localized application of pressure to the femoral region.

To effectively transmit the actuating force evenly to the entire femoral region, it is necessary to increase the rigidity of the femoral support member and have the actuating force of the power actuator to be transmitted evenly to the entire femoral support member. However, in such an exoskeletal walking assistance device, it is not possible to precisely coincide the point of attachment between the power actuator/power transmitting arm and the body with the pivot center (articulation joint) of the body, and this inevitably causes a relative movement between the support member and body. In other words, increasing the rigidity of the femoral support member limits the freedom of movement of the wearer.

To increase the rigidity of the support member without obstructing the freedom of movement of the wearer, a relative movement between the support member and body must be accommodated by using a sliding mechanism or link mechanism between the power actuator and femoral support member. This leads to an increase in the complexity in the structure of the device and adds to the weight of the device.

An assistance device of this kind is designed for a person having limited bodily capabilities, and it is therefore not desirable if the femoral support member is not easy to wear or is too heavy.

The present invention was made in view of such problems of the prior art, and has a primary object to provide a walking assistance device equipped with a femoral support member that can favorably accommodate a relative movement between the support member and a human body while increasing the rigidity of a power transmitting portion as well as the femoral support member.

JP 2013 220102A discloses a walking assistance device having belts for securing an assist force transmitting arm to a femoral region of the wearer.

### MEANS TO ACCOMPLISH THE TASKS

According to the present invention, such an object can be accomplished by providing a walking assistance device, comprising a hip support member designed to be secured to a hip of a wearer, a power actuator secured to the hip support member and having a drive shaft, an assist force transmitting arm extending radially from the drive shaft and a femoral support member for securing a free end of the assist force transmitting arm to a femoral region of the wearer, characterized in that: the femoral support member is designed to engage the femoral region via a pad including a bag and rolling granules filled in the bag, the femoral support member further comprises a stay that supports the pad on a free end of the assist force transmitting arm and is designed to at least partly surround the femoral region of the wearer, and in that the femoral support member comprises a front support plate attached to the stay so as to oppose a front face of the femoral region and a rear support plate attached to the stay so as to oppose a rear face of the femoral region, each of the support plates being provided with a pad so as to engage a corresponding part of the femoral region via the pad.

Thereby, the fact that the femoral region of the wearer is allowed to make a sliding contact with the femoral support member. This accommodates the relative movement between the support member and body owing to the disagreement between the axis of body movement (center of an articulate joint) and the point at which the power actuator/power transmitting arm engages the wearer's body. It further permits the flexing movement and twisting movement of the lower limb, and would not prevent natural movement of the lower limb. Therefore, the present invention is highly effective in minimizing the discomfort to the wearer.

If the assist force transmitting arm consists of a member that is substantially rigid with respect to a rotational direction thereof, the distance between the power actuator secured adjacent to the hip joint and the femoral support member so that the femoral support member would not shift during use.

Since the femoral support member comprises a stay that supports the pad on a free end of the assist force transmitting member and at least partly surrounds the femoral region of the wearer, the femoral region can be secured to a free end of the assist force transmitting arm in a stable manner. In particular, since the femoral support member comprises a front support plate attached to the stay so as to oppose a front face of the femoral region and a rear support plate attached to the stay so as to oppose a rear face of the femoral region, each of the support plate being provided with a pad so as to engage a corresponding part of the femoral region via the pad, the pressure supporting surface area can be maximized. In view of the anatomy of a human body, it is preferable if the front support plate is vertically longer than the rear support plate and/or the rear support plate is wider than the front support plate.

To favorably accommodate the movement of the femoral region, it is preferable if each support plate is rotatably attached to the stay and/or each support plate is attached to the stay via a spring joint. In particular, it is important to permit a relative sliding movement between the femoral region and femoral support member in a favorable manner. For this purpose, the bag is preferably divided into a plurality of cells each of which is filled with the rolling granules. For the granules to be light in weight and capable rolling in a favorable manner, the rolling granules preferably comprise plastic material. From the view point of comfort, the rolling granules may comprise foamed plastic material so as to be easily deformable.

### BRIEF DESCRIPTION OF THE DRAWINGS

Now the present invention is described in the following with reference to the appended drawings, in which:
Figure 1 is a perspective view showing an overall structure of a walking assistance device embodying the present invention;
Figure 2 is a plan view of the femoral support member;
Figure 3 is a perspective view of the femoral support member; and
Figure 4 is a perspective view of the front support of the femoral support member.

### DETAILED DESCRIPTION OF THE PREFERRED EMBODIMENTS

Now the present invention is described in the following in more detail in terms of a concrete embodiment with reference to the appended drawings.

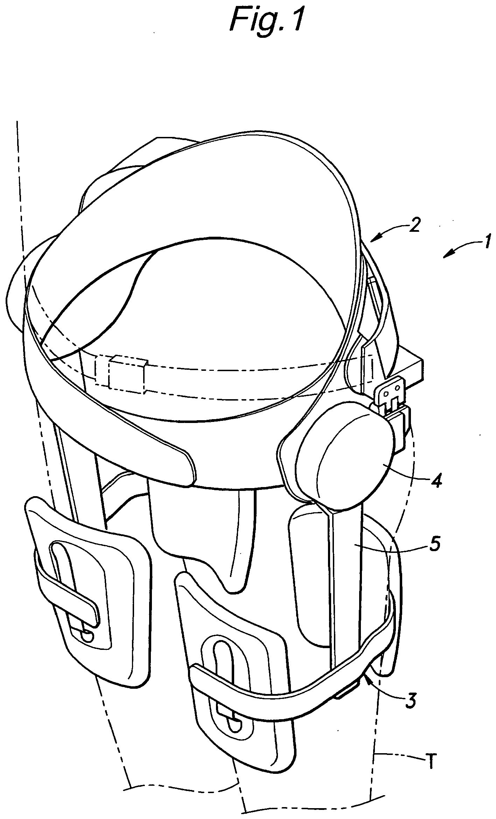

Figure 1 is a simplified structural view of a walking assistance device embodying the present invention. This walking assistance device 1 comprises a hip support member 2, a pair of femoral support members 3 and an actuator 4 attached to the hip support member 2 for producing an assist power for each femoral support members 3. Each actuator 4 is secured to a side of a hip joint of a wearer by securing the hip support member 2 to the hip portion of the wearer. By wearing the femoral support member 3 on a femoral region T of the wearer, the assist force which is applied to a free end of a torque arm 5 extending from an output shaft of the actuator 4 along an outer side of the femoral region T is transmitted to the femoral region T.

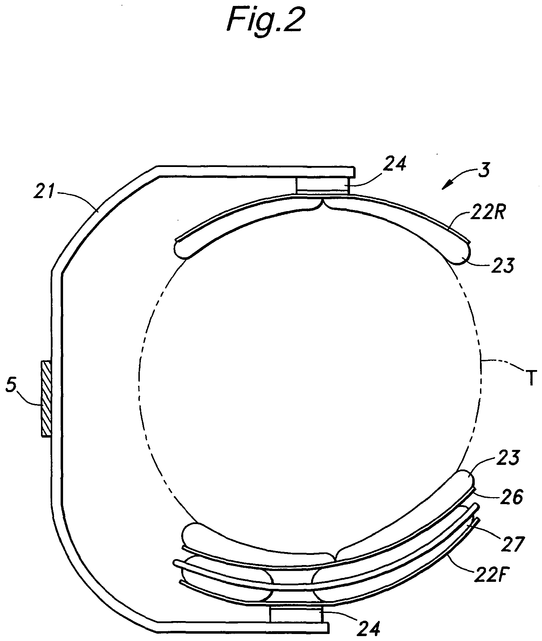

Referring to Figure 2, each femoral support member 3 connected to the free end of the torque arm 5 comprises a stay 21 consisting of a flat bar bent into the shape of letter-U as seen in plan view, a pair of support plates 22F and 22R each made of a thin plate member and attached to the inner face of a corresponding one of the two opposing ends of the stay 21 and a pair of pads 23 attached to the opposing inner surfaces of the two support plates 22F and 22R, respectively, or the surfaces that engage the femoral region T. The stay 21 is substantially rigid with respect to a rotational direction thereof but is somewhat flexible in the inward and outward directions. The outer surface of each support plate 22F and 22R is connected to a flexible connecting plate 24 (that serves the dual purposes of a hinge and a sheet spring) at an upper and a lower part of the connecting plate 24, and the stay 21 is connected to an intermediate point of the flexible connecting plate 24. The stay 21, support plates 22F and 22R and flexible connecting plate 24 are made of plastic material reinforced with fibers such as carbon fibers and Kevler fibers for the purpose of achieving both a high rigidity and a light weight. The joint between each torque arm 5 and the corresponding stay 21 as well as the joint between the stay 21 and each support plate 22F and 22R may comprise a threaded bolt and nut that are used in combination with a plurality of holes for passing the bolt so that the position of the joint may be adjusted to the build of the wearer by selecting one of the holes.

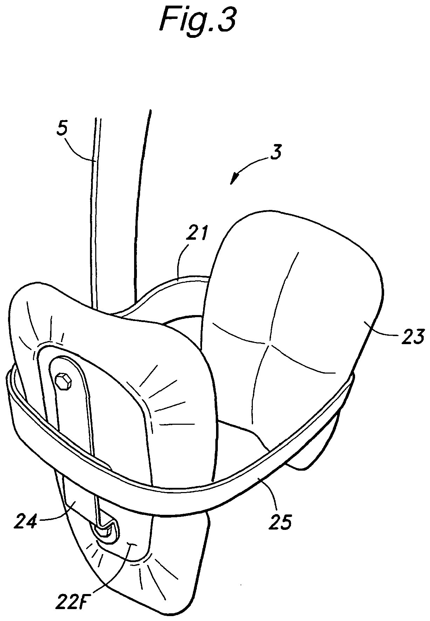

The support plates 22F and 22R are configured so as to interpose the femoral region T between the opposing surfaces thereof via the pads 23 from front and rear. The front support plate 22F engaging the front surface of the femoral region T is vertically more elongated than the rear support plate 22R engaging the rear surface of the femoral region T, and the rear support plate 22R is wider than the front support plate 22F. As shown in Figure 3, the femoral support member 3 is secured to the femoral region T by wrapping a web belt 25 around the femoral region T with the support plates 22F and 22R interposed between them, and joining the two ends of the web belt 25 by using a planar fastener.

The rear support plate 22R engages the hamstring of the wearer that includes a femoral biceps, and the front support plate 22F engages the femoral quadriceps of the wearer. To avoid the rear support plate 22R from interfering with the back of the knee of the wearer when the wearer squats, the rear support plate 22R has a raised lower edge and is shorter by the front support plate F by the corresponding length. Because the muscular force of the femoral quadriceps is about 1.6 times greater than that of the hamstring when supporting the human weight, the efficiency of the force transmission from the torque arm 5 can be increased by increasing the vertical dimension of the front support plate 22F so as to increase the load supporting capability and arranging the central path of force transmission in parallel with the femoral bone.

The pad 23 that engages the femoral region T consists of a bag made by sewing sheet material and filled with foamed plastic granules (beads).

The sheet material for the bag is desired to be pliant and low in friction. In terms of a capability to retain shape, thermoplastic plastic such as nylon, polyester, polypropylene is preferred. In particular, non-woven fabric, knit fabric and woven fabrics made of fibers of such plastic material are preferred. Absorption property is also important in view of the inevitable sweating of the wearer, but as this pad 23 is typically worn over a garment worn by the wearer, the material is required to be highly air permeable at least.

The beads filled in the bag may be readily formed by shaping plastic material into granules while the plastic material is being foamed or by breaking lumps of suitably foamed plastic material into fine particles. The plastic material may consist of those that have a molecular structure free from polar groups and are low in friction, such as polystyrene and polypropylene. If the particle size is too small, comfort to the wearer is impaired because the particles would not readily undergo elastic deformation and the wearer may feel the particles to be too hard. If the particle size is too great, the bag demonstrates an irregular outer contour, and this impairs the appearance of the pad 23. Based on such considerations, the particle size is desired to be in the range of 0.2 mm to 2mm.

If the amount of the beads that fill the bag is too small, the bag is unable to properly conform to the outer surface of the femoral region T. Therefore, the amount of the beads that fill the bag should be such that the beads can freely move inside the bag and a layer of beads having a suitable thickness may be always present between the femoral region T and pad 23 when the pad 23 is pressed against the femoral region T.

Even though the beads are extremely light in weight, the beads tend to sink downward under their own weight when the pad 23 is not worn by the wearer. It is therefore preferable to divide the bag into a number of small cells by sewing so that the beads may be favorably distributed over the entire bag as illustrated in Figure 3. Thereby, when the femoral support member 3 is worn on the femoral region T, the beads that may be settling in a lower part of each cell of the bag may move upward so as to conform to the shape of the femoral region T of the wearer and move from places that are subjected to relatively high pressures to places that are subjected to relatively low pressures so that the entire pad 23 is enabled to evenly engage the femoral region T. In particular, even when the wearer squats, the femoral region T is enabled to contact the two support plates 22F and 22R over a large contact surface area, and the resulting even distribution of the contact pressure ensures a comfort to the wearer.

When there is a relative movement between the femoral support member 3 and femoral region T owing to the movement of the femoral region T, because the beads inside the pad 23 are in rolling contact with one another, the beads can move freely within the bag. Therefore, even when the pad 23 is in close contact with the femoral region T, the friction between the femoral region T and each of the support plates 22F and 22R is reduced by the rolling of the beads so that the relative movement between the femoral support member 3 and femoral region T can be favorably accommodated. Therefore, the movements of the femoral region T, in particular the flexing movement and twisting movement of the femoral region T, can be effected without obstruction.

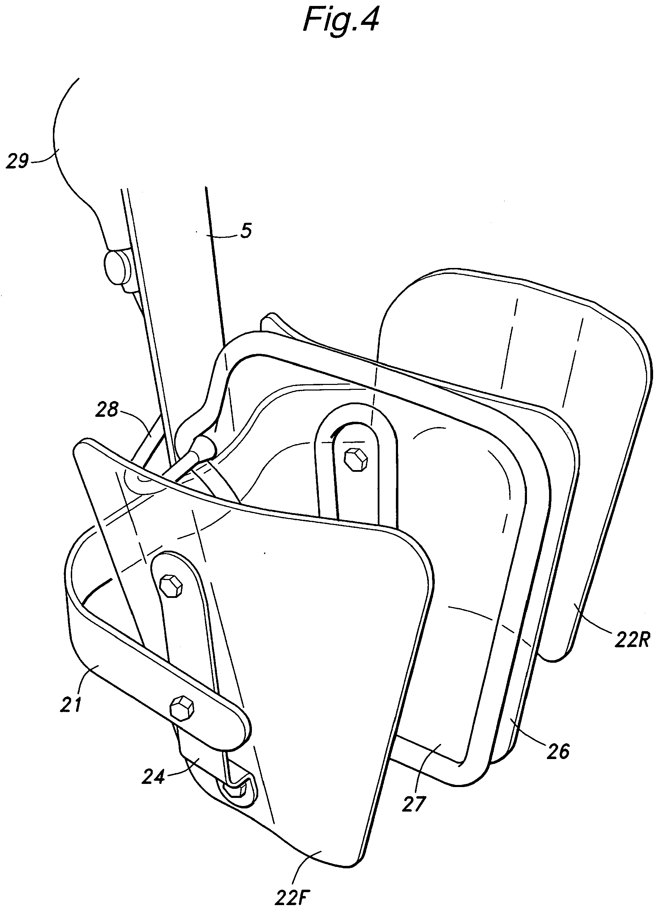

To the inner surface of the front support plate 22F is secured an air pad 27 by using a backup plate 26 having a substantially same shape as the front support plate 22F. This air pad 27 consists of a flattened blister made of soft elastomer material, and is provided with a part that is connected to a hand pump 29 via an air hose 28 as shown in Figure 4. Therefore, by pumping air into the air pad 27 by using the hand pump 29 after fastening the air belt 25, the femoral support member 3 can be even more firmly secured to the femoral region T. In this manner, the femoral support member 3 can be secured to the femoral region T with the benefit of the resiliency of the flexible connecting plate 24 and air pad 27.

The air cushioning action of the air pad 27 allows the air pad 27 to deform to such an extent as to accommodate deformation of the outer surface of the femoral region T owing to the swelling of the muscles such as when the user squats. Also, the securing force of the support member 3 can be readily adjusted by pumping air into and out of the air pad 27. These factors contribute to making the femoral support member 3 comfortable to wear. Because the femoral support member 3 can accommodate a large range of differences in the build of the user by using a simple structure, the femoral support member 3 can fit almost any person if it comes with a small number of different sizes such as S, M and L. Therefore, the femoral support member can be mass produced so that the manufacturing cost can be reduced as compared with the conventional femoral support member which is custom made for the build of each particular user.

Although the present invention has been described in terms of a preferred embodiment thereof, it is obvious to a person skilled in the art that various alterations and modifications are possible without departing from the scope of the present invention which is set forth in the appended claims.

**GLOSSARY**

| | | | |
|---|---|---|---|
| 1 | walking assistance device | 3 | femoral support member |
| 4 | actuator | 5 | torque arm |
| 23 | pad | T | femoral region |

## Claims

1. A walking assistance device (1), comprising a hip support member (2) designed to be secured to a hip of a wearer, a power actuator (4) secured to the hip support member (2) and having a drive shaft, an assist force transmitting arm (5) extending radially from the drive shaft and a femoral support member (3) for securing a free end of the assist force transmitting arm to a femoral region (T) of the wearer, **characterized in that**:
the femoral support member (3) is designed to engage the femoral region via a pad (23) including a bag and rolling granules filled in the bag, the femoral support member (3) further comprises a stay (21) that supports the pad (23) on a free end of the assist force transmitting arm (5) and is designed to at least partly surround the femoral region (T) of the wearer, and **in that** the femoral support member (3) comprises a front support plate (22F) attached to the stay (21) so as to oppose a front face of the femoral region and a rear support plate (22R) attached to the stay (21) so as to oppose a rear face of the femoral region, each of the support plates (22F, 22R) being provided with a pad (23) so as to engage a corresponding part of the femoral region via the pad (23).

2. A walking assistance device (1) according to claim 1, wherein the assist force transmitting arm (5) consists of a member that is substantially rigid with respect to a rotational direction thereof.

3. A walking assistance device (1) according to claim 1 or 2, wherein the front support plate (22F) is vertically longer than the rear support plate (22R).

4. A walking assistance device (1) according to claim 1, 2 or 3, wherein the rear support plate (22R) is wider than the front support plate (22F).

5. A walking assistance device (1) according to any preceding claim, wherein each support plate (22F, 22R) is rotatably attached to the stay (21).

6. A walking assistance device (1) according to any preceding claim, wherein each support plate (22F, 22R) is attached to the stay (21) via a spring joint.

7. A walking assistance device (1) according to any preceding claim, wherein the bag is divided into a plurality of cells each of which is filled with the rolling granules.

8. A walking assistance device (1) according to claim 7, wherein the rolling granules comprise plastic material.

9. A walking assistance device (1) according to claim 8, wherein the rolling granules comprise foamed plastic material.

10. A walking assistance device (1) according to claim 7, wherein the rolling granules comprise easily deformable material.

## Patentansprüche

1. Gehhilfevorrichtung (1), umfassend ein Hüftstützglied (2), das dazu gestaltet ist, an einer Hüfte eines Trägers befestigt zu werden, ein Kraftstellglied (4), das am Hüftstützglied (2) befestigt ist und eine Antriebswelle aufweist, einen Hilfskraftübertragungsarm (5), der radial von der Antriebswelle verläuft, und ein Oberschenkelstützglied (3) zum Befestigen eines freien Endes des Hilfskraftübertragungsarms an einem Oberschenkelbereich (T) des Trägers, **dadurch gekennzeichnet, dass**:
das Oberschenkelstützglied (3) dazu gestaltet ist, über ein Polster (23), das einen Beutel und in den Beutel gefüllte Rollkörnchen enthält, in den Oberschenkelbereich einzugreifen, wobei das Oberschenkelstützglied (3) ferner eine Strebe (21) umfasst, die das Polster (23) an einem freien Ende des Hilfskraftübertragungsarms (5) stützt und dazu gestaltet ist, den Oberschenkelbereich (T) des Trägers zumindest teilweise zu umgeben, und dass das Oberschenkelstützglied (3) eine vordere Stützplatte (22F), die derart an der Strebe (21) angebracht ist, dass sie einer Vorderfläche des Oberschenkelbereichs gegenüberliegt, und eine hintere Stützplatte (22R) umfasst, die derart an der Strebe (21) angebracht ist, dass sie der Rückfläche des Oberschenkelbereichs gegenüberliegt, wobei jede der Stützplatten (22F, 22R) mit einem Polster (23) versehen ist, um über das Polster (23) in einen entsprechenden Teil des Oberschenkelbereichs einzugreifen.

2. Gehhilfevorrichtung (1) nach Anspruch 1, wobei der Hilfskraftübertragungsarm (5) aus einem Glied besteht, das bezüglich einer Drehrichtung davon im Wesentlichen starr ist.

3. Gehhilfevorrichtung (1) nach einem der Ansprüche 1 oder 2, wobei die vordere Stützplatte (22F) vertikal länger als die hintere Stützplatte (22R) ist.

4. Gehhilfevorrichtung (1) nach einem der Ansprüche 1, 2 oder 3, wobei die hintere Stützplatte (22R) breiter als die vordere Stützplatte (22F) ist.

5. Gehhilfevorrichtung (1) nach einem der vorhergehenden Ansprüche, wobei jede Stützplatte (22F, 22R) drehbar an der Strebe (21) angebracht ist.

6. Gehhilfevorrichtung (1) nach einem der vorhergehenden Ansprüche, wobei jede Stützplatte (22F, 22R) über ein Federgelenk an der Strebe (21) angebracht ist.

7. Gehhilfevorrichtung (1) nach einem der vorhergehenden Ansprüche, wobei der Beutel in mehrere Zellen aufgeteilt ist, die jede mit Rollkörnchen gefüllt sind.

8. Gehhilfevorrichtung (1) nach Anspruch 7, wobei die Rollkörnchen Kunststoffmaterial umfassen.

9. Gehhilfevorrichtung (1) nach Anspruch 8, wobei die Rollkörnchen Schaumkunststoffmaterial umfassen.

10. Gehhilfevorrichtung (1) nach Anspruch 7, wobei die Rollkörnchen leicht verformbares Material umfassen.

## Revendications

1. Dispositif d'aide à la marche (1), comprenant un membre de support de hanche (2) conçu pour être fixé à une hanche d'un porteur, un actionneur de puissance (4) fixé au membre de support de hanche (2) et ayant un manche de conduite, un bras de transmission de force d'assistance (5) s'étendant radialement du manche de conduite et un membre de support fémoral (3) pour fixer une extrémité libre du bras de transmission de force d'assistance à une région fémorale (T) du porteur, **caractérisé en ce que** :
le membre de support fémoral (3) est conçu pour engager la région fémorale via un coussinet (23) incluant un sac et des granulés de roulement remplis dans le sac, le membre de support fémoral (3) comprend en outre un appui (21) qui supporte le coussinet (23) sur une extrémité libre du bras de transmission de force d'assistance (5) et est conçu pour entourer au moins en partie la région fémorale (T) du porteur, et **en ce que** le membre de support fémoral (3) comprend une plaque de support frontale (22F) attachée à l'appui (21) de façon à opposer une face frontale de la région fémorale et une plaque de support arrière (22R) attachée à l'appui (21) de façon à opposer une face arrière de la région fémorale, chacune des plaques de support (22F, 22R) étant pourvues avec un coussinet (23) de façon à engager une partie correspondante de la région fémorale via le coussinet (23).

2. Dispositif d'aide à la marche (1) selon la revendication 1, dans lequel le bras de transmission de force d'assistance (5) consiste en un membre qui est essentiellement rigide par rapport à une direction rotationnelle de celui-ci.

3. Dispositif d'aide à la marche (1) selon a revendication 1 ou 2, dans lequel la plaque de support frontale (22F) est verticalement plus longue que la plaque de support arrière (22R).

4. Dispositif d'aide à la marche (1) selon la revendication 1, 2 ou 3, dans lequel la plaque de support arrière (22R) est plus large que la plaque de support frontale (22F).

5. Dispositif d'aide à la marche (1) selon l'une quelconque des revendications précédentes, dans lequel chaque plaque de support (22F, 22R) est attachée de manière rotative à l'appui (21).

6. Dispositif d'aide à la marche (1) selon l'une quelconque des revendications précédentes, dans lequel chaque plaque de support (22F, 22R) est attachée à l'appui (21) via un joint à ressort.

7. Dispositif d'aide à la marche (1) selon l'une quelconque des revendications précédentes, dans lequel le sac est divisé en une pluralité de cellules dont chacune est remplie avec les granulés de roulement.

8. Dispositif d'aide à la marche (1) selon la revendication 7, dans lequel les granulés de roulement comprennent du matériau plastique.

9. Dispositif d'aide à la marche (1) selon la revendication 8, dans lequel les granulés de roulement comprennent du matériau plastique moussé.

10. Dispositif d'aide à la marche (1) selon la revendication 7, dans lequel les granulés de roulement comprennent du matériau facilement déformable.
